# EUROPEAN PATENT APPLICATION

(11) **EP 4 534 080 A1**
(43) Date of publication of application: **09.04.2025**
(21) Application number: 23815582.4
(22) Date of filing: 06.04.2023
(51) Int. Cl.: A61K 31/085, A23L 33/10, A61K 35/618, A61P 1/00, A61P 39/02, A61P 39/06, A61P 43/00

(54) **AGENT FOR PROMOTING DECREASE IN 8-OHDG CONCENTRATION, AGENT FOR PROMOTING DECREASE IN MALONDIALDEHYDE (MDA) CONCENTRATION, AGENT FOR PROMOTING INCREASE IN ZIP4 CONCENTRATION, OR AGENT FOR PROMOTING INCREASE IN SERUM ZINC CONCENTRATION IN JEJUNUM**

(30) Priority: 02.06.2022 JP 2022090145
(71) Applicant: Watanabe Oyster Laboratory Co., Ltd., Hachioji-Shi Tokyo 192-0154 (JP)
(72) Inventor: WATANABE Mitsugu, Hachioji-shi, Tokyo 192-0154 (JP); WATANABE Hideaki, Hachioji-shi, Tokyo 192-0154 (JP)
(74) Representative: Plougmann Vingtoft a/s
(86) International application number: PCT/JP2023/014234
(87) International publication number: WO 2023/233820

(57) **Abstract**

[Problem]

It is an object of the present invention to provide an MDA concentration decrease accelerator, an 8-OHdG concentration decrease accelerator, a ZIP4 concentration increase accelerator, or a serum zinc concentration increase accelerator for the jejunum that can accelerate a decrease in MDA concentration, a decrease in 8-OHdG concentration, an increase in ZIP4 concentration, and an increase in serum zinc concentration for the jejunum to improve an oxidative stress state.

[Solution]

The present invention includes 3,5-dihydroxy-4-methoxybenzyl alcohol as an active ingredient and has an 8-OHdG concentration decrease accelerative action of decreasing a jejunal 8-OHdG concentration.

## Description

### TECHNICAL FIELD

The present invention relates to an 8-OHdG concentration decrease accelerator, an MDA concentration decrease accelerator, a ZIP4 concentration increase accelerator, and a serum zinc concentration increase accelerator for the jejunum.

### BACKGROUND ART

The inventors confirmed a significant increase in serum zinc concentration by administering a Pacific oyster soft body extraction fraction (for example, a fraction obtained by immersing oyster meat in an extraction liquid to extract various components from the oyster meat) to patients with type 2 diabetes, for example, for 12 weeks.

However, a mixed fraction of Pacific oyster soft body extraction precipitation fraction and supernatant fraction (for example, a precipitation fraction precipitated and a supernatant fraction of the supernatant obtained after immersing oyster meat in an extraction liquid and stirring the extraction liquid into which various components were extracted from the oyster meat) did not contain zinc enough to significantly increase the serum zinc concentration. Therefore, the inventors were confused about the mechanism that caused this result.

Therefore, the inventors focused on verifying a zinc absorption mechanism in the intestinal tract and investigated the effect of the Pacific oyster soft body extraction fraction on the zinc absorption mechanism in the intestinal tract.

Zinc is absorbed in the duodenum and jejunum, but it is mainly absorbed in the jejunum. In the absorption of zinc, ZIP4, a zinc transporter in the intestinal tract, is considered to play an essential role.

Conventionally, it has been reported that the expression level of ZIP4 in the intestinal tract increases in a zinc-deficient state. Thus, the present inventors observed the increase in ZIP4 concentration in the duodenum of a rat under low-zinc dietary administration and were able to obtain the same result as the conventional report that "the expression level of ZIP4 in the intestinal tract increases in a zinc-deficient state."

However, in the jejunum under the low-zinc dietary administration, a decrease in the ZIP4 concentration was observed. Changes in the expression levels of ZIP4 concentration in the rat that took in the zinc-deficient diet may be due not only to differences in the degree of zinc deficiency but also to differences in the regions of the intestinal tract.

On one hand, as an interesting report, in corn-based roots and shoots susceptible to zinc deficiency, an H2O2 concentration increased dramatically, and the expression of ZIP4 transporter genes did not increase.

On the other hand, in corn-based roots and shoots having resistance against cultivation in soil with low-zinc content, the expression of the ZIP4 transporter genes increased, and it has been reported that antioxidant protection influences the increase in the expression of the ZIP4 transporter genes.

That is, it was confirmed with corn roots that the expression of the ZIP4 transporter genes is influenced by an oxidative state.

In the present invention, it is predicted that a decrease in superoxide dismutase (SOD) activity induced by a decrease in jejunal zinc concentration due to the low-zinc dietary administration results in an increase in reactive oxygen in the rat jejunum, and a phenomenon related to the increase occurs. That is, it is predicted that an oxidative stress state, which is indicated by an increase in jejunal MDA concentration, an increase in 8-OHdG concentration, a decrease in ZIP4 concentration, and a decrease in serum Zn concentration, will occur.

Hence, the present inventors verified for the oxidative stress state what changes occur to the oxidative stress state by administering, to the rat, respective fractions, that is, a precipitation fraction and a supernatant fraction, of the Pacific oyster soft body extraction fraction having an antioxidant action, components extracted from the Pacific oyster soft body portion, especially 3,5-dihydroxy-4-methoxybenzyl alcohol, and further, synthesized 3,5-dihydroxy-4-methoxybenzyl alcohol (DHMBA). In other words, the present inventors verified whether or not a decrease in MDA concentration, a decrease in 8-OHdG concentration, an increase in the ZIP4 concentration, and an increase in serum zinc concentration are accelerated for the jejunum due to an improvement of the oxidative stress state by administering 3,5-dihydroxy-4-methoxybenzyl alcohol.

Patent Document 1: JP-A-2016-042825

### DISCLOSURE OF THE INVENTION

### PROBLEMS TO BE SOLVED BY THE INVENTION

It is an object of the present invention to provide an MDA concentration decrease accelerator, an 8-OHdG concentration decrease accelerator, a ZIP4 concentration increase accelerator, or a serum zinc concentration increase accelerator for the jejunum that can accelerate a decrease in MDA concentration, a decrease in 8-OHdG concentration, an increase in ZIP4 concentration, and an increase in serum zinc concentration for the jejunum to improve an oxidative stress state. The acceleration of the decrease in MDA concentration, the decrease in 8-OHdG concentration, the increase in ZIP4 concentration, and the increase in serum zinc concentration was verified for an oxidative stress state by administering, to a rat, components extracted from respective fractions, that is, a precipitation fraction and a supernatant fraction, of a Pacific oyster soft body extraction fraction having an antioxidant action, especially 3,5-dihydroxy-4-methoxybenzyl alcohol, and further, synthesized 3,5-dihydroxy-4-methoxybenzyl alcohol (DHMBA).

### SOLUTIONS TO THE PROBLEMS

The present invention includes 3,5-dihydroxy-4-methoxybenzyl alcohol as an active ingredient and has an 8-OHdG concentration decrease accelerative action of decreasing a jejunal 8-OHdG concentration.

Alternatively, the present invention includes 3,5-dihydroxy-4-methoxybenzyl alcohol extracted from oyster meat as an active ingredient and has an 8-OHdG concentration decrease accelerative action of decreasing a jejunal 8-OHdG concentration.

Alternatively, the present invention includes a supernatant fraction as an active ingredient, which is obtained by stirring an extraction liquid extracted from oyster meat and then taking out a supernatant extract, and has an 8-OHdG concentration decrease accelerative action of decreasing a jejunal 8-OHdG concentration.

Alternatively, the present invention includes a precipitation fraction as an active ingredient, which is precipitated after stirring an extraction liquid extracted from oyster meat, and has an 8-OHdG concentration decrease accelerative action of decreasing a jejunal 8-OHdG concentration.

Alternatively, the present invention includes synthesized 3,5-dihydroxy-4-methoxybenzyl alcohol as an active ingredient and has an 8-OHdG concentration decrease accelerative action of decreasing a jejunal 8-OHdG concentration.

Alternatively, the present invention includes 3,5-dihydroxy-4-methoxybenzyl alcohol as an active ingredient and has an MDA concentration decrease accelerative action of decreasing a jejunal MDA concentration.

Alternatively, the present invention includes 3,5-dihydroxy-4-methoxybenzyl alcohol extracted from oyster meat as an active ingredient and has an MDA concentration decrease accelerative action of decreasing a jejunal MDA concentration.

Alternatively, the present invention includes a supernatant fraction as an active ingredient, which is obtained by stirring an extraction liquid extracted from oyster meat and then taking out a supernatant extract, and has an MDA concentration decrease accelerative action of decreasing a jejunal MDA concentration.

The present invention includes a precipitation fraction as an active ingredient, which is precipitated after stirring an extraction liquid extracted from oyster meat, and has an MDA concentration decrease accelerative action of decreasing a jejunal MDA concentration.

Alternatively, the present invention includes synthesized 3,5-dihydroxy-4-methoxybenzyl alcohol as an active ingredient and has an MDA concentration decrease accelerative action of decreasing a jejunal MDA concentration.

Alternatively, the present invention includes 3,5-dihydroxy-4-methoxybenzyl alcohol as an active ingredient and has a ZIP4 concentration increase accelerative action of increasing a jejunal ZIP4 concentration.

Alternatively, the present invention includes 3,5-dihydroxy-4-methoxybenzyl alcohol extracted from oyster meat as an active ingredient and has a ZIP4 concentration increase accelerative action of increasing a jejunal ZIP4 concentration.

Alternatively, the present invention includes a supernatant fraction as an active ingredient, which is obtained by stirring an extraction liquid extracted from oyster meat and then taking out a supernatant extract, and has a ZIP4 concentration increase accelerative action of increasing a jejunal ZIP4 concentration.

Alternatively, the present invention includes a precipitation fraction as an active ingredient, which is precipitated after stirring an extraction liquid extracted from oyster meat, and has a ZIP4 concentration increase accelerative action of increasing a jejunal ZIP4 concentration.

Alternatively, the present invention includes synthesized 3,5-dihydroxy-4-methoxybenzyl alcohol as an active ingredient and has a ZIP4 concentration increase accelerative action of increasing a jejunal ZIP4 concentration.

Alternatively, the present invention includes 3,5-dihydroxy-4-methoxybenzyl alcohol as an active ingredient and has an action of inducing an increase in serum zinc concentration.

Alternatively, the present invention includes 3,5-dihydroxy-4-methoxybenzyl alcohol extracted from oyster meat as an active ingredient and has an action of inducing an increase in serum zinc concentration.

Alternatively, the present invention includes synthesized 3,5-dihydroxy-4-methoxybenzyl alcohol as an active ingredient and has an action of inducing an increase in serum zinc concentration.

Alternatively, the present invention includes a supernatant fraction containing DHMBA as an active ingredient, which is obtained by stirring an extraction liquid extracted from oyster meat and then taking out a supernatant extract, and has an action of inducing an increase in serum zinc concentration.

Alternatively, the present invention includes a precipitation fraction containing DHMBA as an active ingredient, which is precipitated after stirring an extraction liquid extracted from oyster meat, and has an action of inducing an increase in serum zinc concentration.

### EFFECTS OF THE INVENTION

The present invention provides an excellent effect of improving an oxidative stress state and accelerating a decrease in MDA concentration, a decrease in 8-OHdG concentration, an increase in ZIP4 concentration, and an increase in serum zinc concentration for the jejunum. The effect was verified for an oxidative stress state by administering, to a rat, components extracted from respective fractions, that is, a precipitation fraction and a supernatant fraction, of a Pacific oyster soft body extraction fraction having an antioxidant action, especially 3,5-dihydroxy-4-methoxybenzyl alcohol, and further, synthesized 3,5-dihydroxy-4-methoxybenzyl alcohol (DHMBA).

### BRIEF DESCRIPTION OF THE DRAWINGS

FIG. 1 is an explanatory drawing of a jejunal SOD concentration under a standard diet and a low-zinc diet.
FIG. 2 is an explanatory drawing of a jejunal 8-OHdG concentration for standard diet, low-zinc diet, and low-zinc diet + supernatant fraction groups.
FIG. 3 is an explanatory drawing of the jejunal 8-OHdG concentration for standard diet, low-zinc diet, and low-zinc diet + precipitation fraction groups.
FIG. 4 is an explanatory drawing of the jejunal 8-OHdG concentration for standard diet, low-zinc diet, and low-zinc diet + synthetic DHMBA groups.
FIG. 5 is an explanatory drawing of a jejunal MDA concentration for the standard diet, low-zinc diet, and low-zinc diet + supernatant fraction groups.
FIG. 6 is an explanatory drawing of a jejunal ZIP4 concentration for the standard diet, low-zinc diet, and low-zinc diet + supernatant fraction groups.
FIG. 7 is an explanatory drawing of the jejunal ZIP4 concentration for the standard diet, low-zinc diet, and low-zinc diet + precipitation fraction groups.
FIG. 8 is an explanatory drawing of the jejunal ZIP4 concentration for the standard diet, low-zinc diet, and low-zinc diet + synthetic DHMBA groups.
FIG. 9 is an explanatory drawing describing the correlation between the MDA concentration and a ZIP concentration in the standard diet, low-zinc diet, and low-zinc diet + supernatant fraction groups.
FIG. 10 is an explanatory drawing describing the correlation between the MDA concentration and the ZIP concentration in the standard diet, low-zinc diet, and low-zinc diet + precipitation fraction groups.
FIG. 11 is an explanatory drawing describing the correlation between the MDA concentration and the ZIP concentration in the standard diet, low-zinc diet, and low-zinc diet + synthetic DHMBA groups.
FIG. 12 is an explanatory drawing describing a serum zinc concentration for the standard diet, low-zinc diet, and low-zinc diet + synthetic DHMBA groups.
FIG. 13 is an explanatory drawing describing the correlation between the jejunal ZIP concentration and the serum zinc concentration in the standard diet, low-zinc diet, and low-zinc diet + synthetic DHMBA groups.
FIG. 14 is an explanatory drawing describing the correlation between the jejunal ZIP concentration and the serum zinc concentration in the standard diet, low-zinc diet, and low-zinc diet + supernatant fraction groups.
FIG. 15 is an explanatory drawing describing the correlation between the jejunal ZIP concentration and the serum zinc concentration in the standard diet, low-zinc diet, and low-zinc diet + precipitation fraction groups.
FIG. 16 is an explanatory drawing describing the correlation between the jejunal MDA concentration and the serum zinc concentration in the standard diet, low-zinc diet, and low-zinc diet + synthetic DHMBA groups.
FIG. 17 is an explanatory drawing describing the correlation between the jejunal MDA concentration and the serum zinc concentration in the standard diet, low-zinc diet, and low-zinc diet + supernatant fraction groups.
FIG. 18 is an explanatory drawing describing the correlation between the jejunal MDA concentration and the serum zinc concentration in the standard diet, low-zinc diet, and low-zinc diet + precipitation fraction groups.

### DESCRIPTION OF PREFERRED EMBODIMENTS

### (EXAMPLES)

The present invention is to manufacture accelerators that contain 3,5-dihydroxy-4-methoxybenzyl alcohol as an active ingredient, accelerate the improvement of an oxidative stress state in the jejunum, and accelerate a decrease in MDA concentration, a decrease in 8-OHdG concentration, an increase in ZIP4 concentration, and an increase in serum zinc concentration and to provide the manufactured various accelerators.

The accelerators are manufactured by immersing oyster meat in an extraction liquid and using 3,5-dihydroxy-4-methoxybenzyl alcohol extracted from the oyster meat immersed in the extraction liquid as an active ingredient. Note that the 3,5-dihydroxy-4-methoxybenzyl alcohol may be synthesized 3,5-dihydroxy-4-methoxybenzyl alcohol.

Then, when the accelerators are manufactured, an animal experiment is conducted to confirm whether the oxidative stress state in the jejunum can improve and whether the decrease in MDA concentration, the decrease in 8-OHdG concentration, the increase in ZIP4 concentration, and the increase in serum zinc concentration can be accelerated.

That is, when a low-zinc diet composed of predetermined components is taken in by a predetermined rat, the activity of superoxide dismutase (SOD) decreases, and reactive oxygen increases in the rat jejunum, causing an increase in the jejunal MDA concentration, an increase in the 8-OHdG concentration, a decrease in the ZIP4 concentration, a decrease in the serum Zn concentration, and the like.

In contrast to this, for example, oyster meat is immersed in an extraction liquid, 3,5-dihydroxy-4-methoxybenzyl alcohol having flowed into the extraction liquid from the immersed oyster meat is extracted, and this is added to a low-zinc diet as a meal for a rat.

Note that it has been confirmed that the 3,5-dihydroxy-4-methoxybenzyl alcohol of the oyster meat having flowed into the extraction liquid differs in quantity and the like in a precipitation fraction precipitated after a lapse of a predetermined time after stirring the extraction liquid and a supernatant fraction of the supernatant obtained after a lapse of the predetermined time after stirring the extraction liquid.

Hence, when 3,5-dihydroxy-4-methoxybenzyl alcohol, which is extracted from oyster meat and has an antioxidant action, and further, synthesized 3,5-dihydroxy-4-methoxybenzyl alcohol (hereinafter also referred to as DHMBA) are administered to the rat in the light of the circumstance described above, how the concentrations of MDA, 8-OHdG, ZIP4, and serum Zn in the rat jejunum vary can be verified and recognized.

Through the verification, excellent efficacy can be confirmed when the respective accelerators are manufactured, and the accelerators that can effectively cope with an oxidative stress state can be manufactured and provided.

Here, an outline of the animal experiment is described.

### (Experimental Method)

Male Slc/SD rats (4 weeks old) were used as experimental animals.

### (Grouping and Diet)

The rats were divided into the following groups, and the diets were as follows.

Here, n indicates the number of rats.

Standard diet group (n = 6): Free intake of a standard diet
(The concentration of zinc in the standard diet: 34.1 mg zinc/kg diet)
Note that the zinc concentration of the standard diet is assumed to be 20 mg zinc/kg diet to 55 mg zinc/kg diet.)
(In addition, the concentration of DHMBA in the standard diet is zero.)
Low-zinc diet group (n = 6): Free intake of a low-zinc diet
(The concentration of zinc in the low-zinc diet: 3.9 mg zinc/kg diet)
Note that the zinc concentration of the low-zinc diet is assumed to be 2.5 mg zinc/kg diet to 10 mg zinc/kg diet.)
(In addition, the concentration of DHMBA in the low-zinc diet is zero.)
Low-zinc diet + supernatant fraction group (n = 6): Low-zinc diet + Pacific oyster extraction supernatant fraction
The zinc concentration of the low-zinc diet is as described above.

In addition, the Pacific oyster extraction supernatant fraction is a fraction obtained by, for example, immersing oyster meat in an extraction liquid, stirring the extraction liquid into which various components are extracted from the oyster meat, and then taking out a supernatant extract on the surface side.

The concentration of DHMBA in the supernatant fraction is 0.96 µg/rat B.W. 100 g.

Note that the DHMBA concentration of the supernatant fraction is assumed to be 0.68 µg/rat B.W. 100 g to 2.38 µg/rat B.W. 100 g.

Low-zinc diet + precipitation fraction group (n = 6): Low-zinc diet + Pacific oyster extraction precipitation fraction
The zinc concentration of the low-zinc diet is as described above.

The Pacific oyster extraction precipitation fraction is a fraction obtained by, for example, immersing oyster meat in an extraction liquid, stirring the extraction liquid into which various components are extracted from the oyster meat, and then taking out a precipitated extract. Note that the precipitation fraction was given by gavage administration, but the concentration of zinc in the fraction is 0.894 µg/day, which is considered to be a negligible zinc amount.

The concentration of DHMBA in the precipitation fraction is 0.38 µg/rat B.W. 100 g.

Note that the DHMBA concentration of the precipitation fraction is assumed to be 0.1 µg/rat B.W. 100 g to 0.5 µg/rat B.W. 100 g.)

Low-zinc diet + synthetic DHMBA group (n = 6): Low-zinc diet + synthetic DHMBA
The zinc concentration of the low-zinc diet is as described above, and the zinc concentration is 3.9 mg zinc/kg diet.

The synthetic DHMBA is not DHMBA extracted from oyster meat but DHMBA produced by chemically synthesizing. Then, the DHMBA concentration is 7.9 mg /rat B.W. 100 g.

Note that the DHMBA concentration of the synthetic DHMBA is assumed to range from 5 mg/rat B.W. 100 g to 15 mg/rat B.W. 100 g depending on the state of production.

### (Test Schedule)

### [1] Preliminary breeding period (7 days)

Test animals were provided with preliminary breeding from the time they were received to the day before the start of regular breeding and acclimated by individual breeding (n = 1/cage). The standard diet was fed during the preliminary breeding period, and grouping was conducted considering the weight at the end date of the preliminary breeding.

### [2] Regular breeding period (7 days)

From the start date of the regular breeding, the standard diet was fed to the standard diet group, and the low-zinc diet was fed to four groups including the low-zinc diet group, the low-zinc diet + precipitation fraction group, the low-zinc diet + supernatant fraction group, and the low-zinc diet + synthetic DHMBA group. They were fed by free intake. On days 5 and 6, 0.5% methyl cellulose solution was given to the standard diet group and the low-zinc diet group by gavage administration. The Pacific oyster extraction supernatant fraction was given to the low-zinc diet + supernatant fraction group by gavage administration. The Pacific oyster extraction precipitation fraction was given to the low-zinc diet + precipitation fraction group by gavage administration. The synthetic DHMBA was given to the low-zinc diet + synthetic DHMBA group by gavage administration. The dosage amount of the test objects was 0.5 mL/100 g B.W. Then, 24 hours after the administration of the test objects on day 6, anatomy was performed to harvest organs.

### (Measurement Items)

The jejunal SOD concentration, MDA concentration, 8-OHdG concentration, ZIP4 concentration, and serum zinc concentration were measured.

### About SOD Concentration

Superoxide dismutase (SOD) is an enzyme that has the power to remove reactive oxygen. SOD is abundant in the mitochondria of cells and has a function of protecting the body from oxidative stress caused by the reactive oxygen.

SOD is an enzyme that converts superoxide into hydrogen peroxide and oxygen, that is, catalyzes the reaction of 2•O2·- + 2H+ → H₂O₂ + O₂. SOD being present despite the fact that originally this reaction proceeds extremely fast even without enzymes indicates that it is important for living organisms to erase superoxide as quickly as possible. In the cytoplasm of eukaryotic organisms, CuZn-SOD containing copper and zinc at the active center is present.

Therefore, as the superoxide dismutase (SOD) concentration decreases, the oxidative stress state increases.

### About MDA Concentration

MDA is malondialdehyde. MDA is one of the lipid peroxidation degradation products and is used as a major marker of lipid peroxidation. The oxidative stress state or the like can be verified as an indicator of lipid peroxidation in cell and tissue samples. An increase in the MDA concentration indicates an increased oxidative stress state.

MDA is produced by hydroxyl radicals (•OH) and the like. When an unsaturated fatty acid is oxidized by the hydroxyl radicals (•OH) and the like, it becomes a lipid peroxidation degradation product, and then, the end product is MDA. When the hydroxyl radicals (•OH) and the like are present in organs at a high concentration, the MDA concentration is high. A decrease in the MDA concentration means a decrease in •OH.

### About 8-OHdG Concentration

8-OHdG is a substance whose guanine base is changed to 8-hydroxy-deoxyguanosine (8-OHdG) by oxidation. DNA is composed of four kinds of bases including adenine, guanine, cytosine, and thymine. DNA is known to be oxidatively damaged by reactive oxygen, and the guanine base is changed to 8-OHdG by oxidation. This 8-OHdG is removed from the gene body during the genetic DNA repair process and excreted into urine through the blood. Furthermore, 8-OHdG is a relatively stable substance that is rapidly excreted in the urine without being metabolized or degraded in living organisms, and therefore, it is used as an excellent biomarker that sensitively reflects biological damage caused by reactive oxygen.

Since dG has the lowest redox potential of the four bases in DNA, it is susceptible to oxidation by reactive oxygen species.

For this reason, 8-OHdG, which is a major oxidation product of dG, sensitively reflects the effects of reactive oxygen species on living organisms. An increase in the 8-OHdG concentration indicates an increased oxidative stress state.

### About ZIP4 Concentration

A zinc transporter functions to maintain zinc homeostasis in living organisms. The zinc transporter ZIP4 (ZIP4: ZRT, IRT-like protein 4) works on the mechanism of zinc absorption in the digestive tract.

ZIP4 is a transmembrane protein that transports zinc from the jejunal cavity to jejunal cells.

### About Serum Zn Concentration

Zinc is an essential mineral in humans, the second most common mineral in living organisms after iron, and is contained in a large amount in bones, muscles, skin, hair, liver, taste buds, testicles, and the like. Its role in living organisms is indispensable for the activity of at least 300 enzymes, and its shortage causes various symptoms, such as taste disorder, dermatitis, hair loss, anemia, stomatitis, diarrhea, male sexual dysfunction, increased susceptibility to infection (immunological deterioration), and osteoporosis. Furthermore, zinc deficiency disorders have been pointed out in many patients with cirrhosis, diabetes, chronic inflammatory bowel disease, and chronic kidney disease.

### (Results and Consideration)

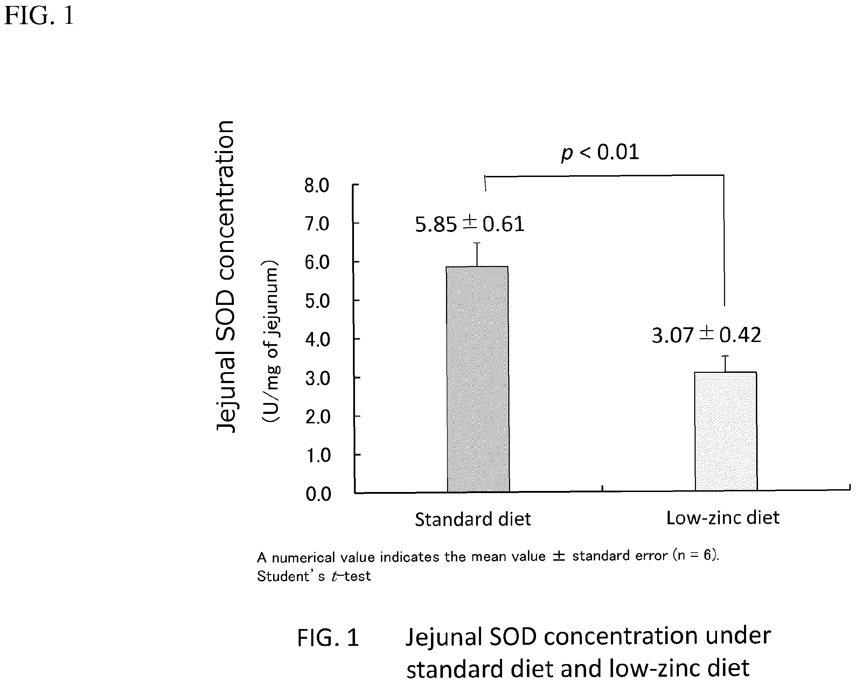

As shown in FIG. 1, the jejunal SOD concentration for the low-zinc diet group of 3.07±0.42 U/mg of jejunum significantly decreased from that for the standard diet group of 5.85±0.61 U/mg of jejunum. Based on this, a decrease in the SOD concentration due to the intake of the low-zinc diet was confirmed. As the superoxide dismutase (SOD) concentration decreases, the oxidative stress state increases.

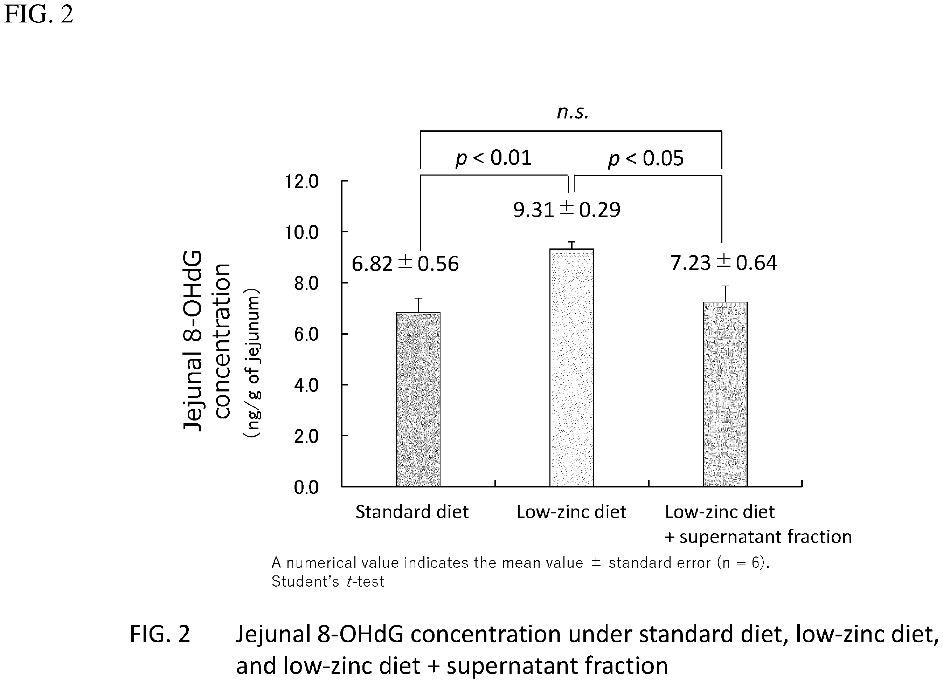

Next, as shown in FIG. 2, the low-zinc diet + supernatant fraction group had a jejunal 8-OHdG concentration of 7.23±0.64, which was significantly lower than the 8-OHdG concentration for the low-zinc diet group of 9.31±0.29. In addition, the 8-OHdG concentration showed no significant difference from the 8-OHdG concentration under the standard diet in spite of the intake of the low-zinc diet and decreased to the same level as that under the standard diet.

Therefore, based on this, a decrease in the 8-OHdG concentration in the jejunum was accelerated by the DHMBA in the supernatant fraction in the low-zinc diet + supernatant fraction group, and accordingly, a DNA antioxidant action was confirmed.

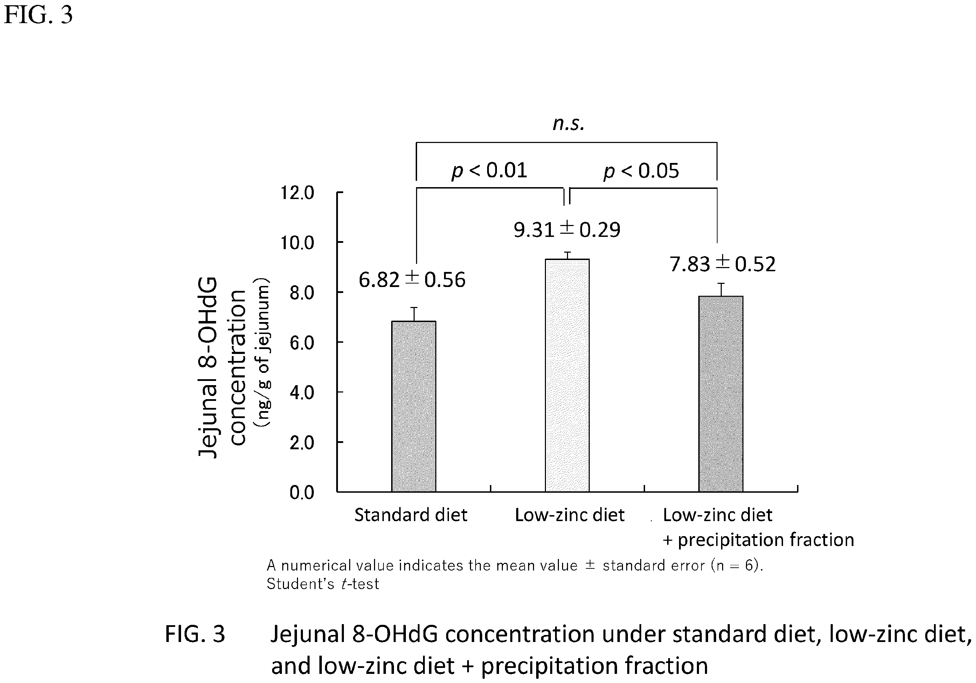

As shown in FIG. 3, the jejunal 8-OHdG concentration for the low-zinc diet group of 9.31±0.29 ng/g of jejunum significantly increased from that for the standard diet group of 6.82±0.56 ng/g of jejunum. This is believed to be due to an increase in reactive oxygen in the jejunum, and based on this, it was confirmed that oxidative stress was induced by the intake of the low-zinc diet.

In contrast to this, the low-zinc diet + precipitation fraction group had an 8-OHdG concentration, which was significantly lower than the 8-OHdG concentration for the low-zinc diet group. In addition, the 8-OHdG concentration showed no significant difference from the 8-OHdG concentration under the standard diet in spite of the intake of the low-zinc diet and decreased to the same level as that under the standard diet. Based on this, a decrease in the 8-OHdG concentration in the jejunum was accelerated by the precipitation fraction in the low-zinc diet + precipitation fraction group, and accordingly, the DNA antioxidant action was confirmed.

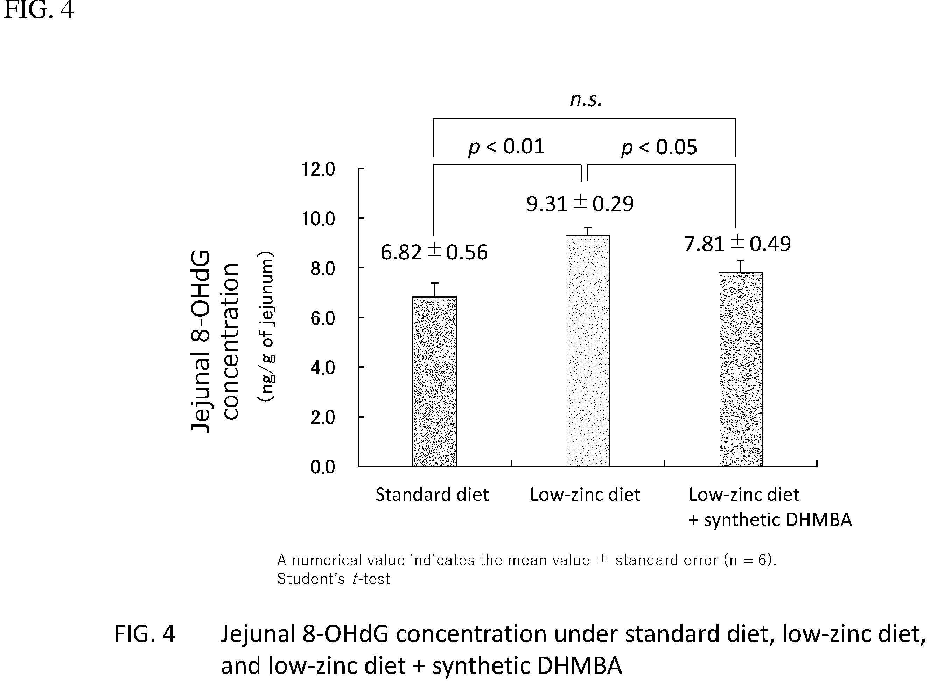

As shown in FIG. 4, the low-zinc diet + synthetic DHMBA group had an 8-OHdG concentration of 7.81±0.49, which was significantly lower than the 8-OHdG concentration for the low-zinc diet group of 9.31±-0.29. In addition, the 8-OHdG concentration showed no significant difference from the 8-OHdG concentration under the standard diet in spite of the intake of the low-zinc diet and decreased to the same level as that under the standard diet.

Therefore, based on this, a decrease in the 8-OHdG concentration in the jejunum was accelerated by the synthetic DHMBA in the low-zinc diet + synthetic DHMBA fraction group, and accordingly, the DNA antioxidant action was confirmed.

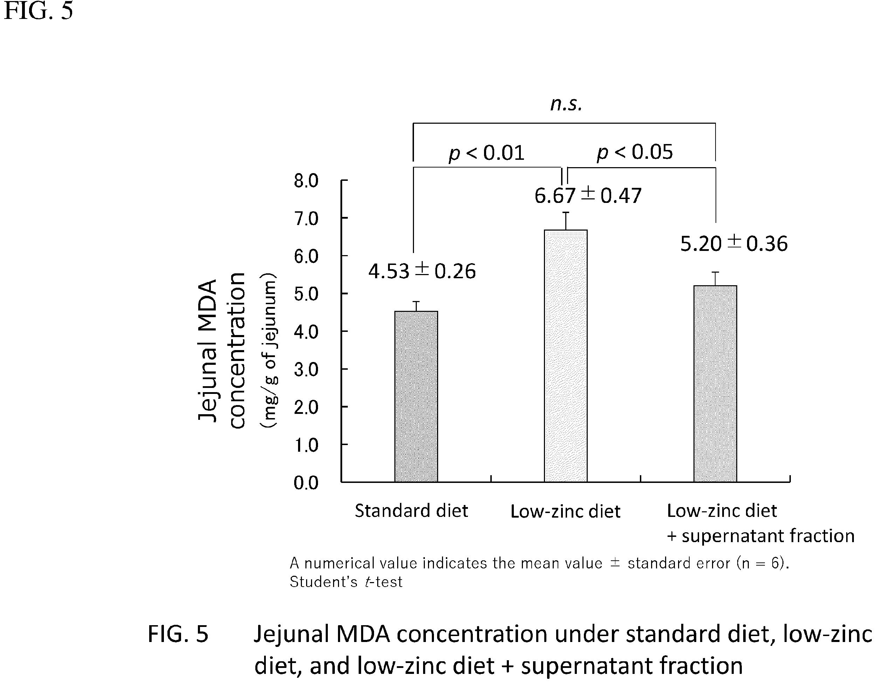

As shown in FIG. 5, the jejunal MDA concentration for the low-zinc diet group of 6.67±0.47 mg/g of jejunum significantly increased from that for the standard diet group of 4.53±0.26 mg/g of jejunum. Based on this, an increase in reactive oxygen (oxidative stress) caused by the decrease in the SOD concentration due to the intake of the low-zinc diet is confirmed.

In contrast to this, the low-zinc diet + supernatant fraction group had an MDA concentration, which was significantly lower than the MDA concentration for the low-zinc diet group. In addition, the MDA concentration showed no significant difference from the MDA concentration under the standard diet in spite of the intake of the low-zinc diet and decreased to the same level as that under the standard diet. Based on this, an antioxidant action against an unsaturated fatty acid in the jejunum by the DHMBA in the supernatant fraction in the low-zinc diet + supernatant fraction group was confirmed.

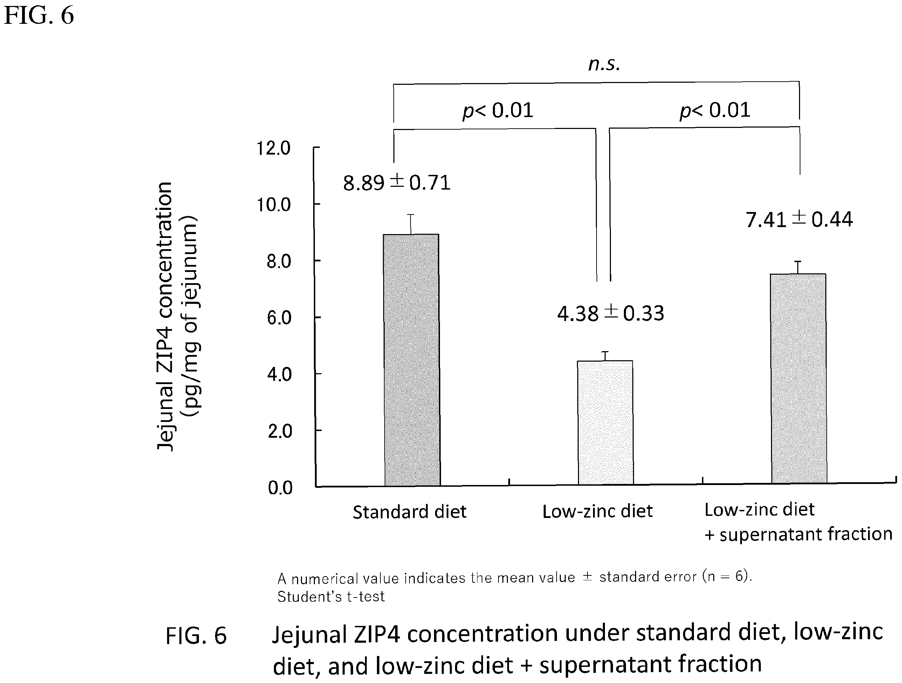

As shown in FIG. 6, the low-zinc diet + supernatant fraction group had a ZIP4 concentration of 7.41±0.44 pg/mg, which was significantly higher than the ZIP4 concentration for the low-zinc diet group of 4.38±0.33 pg/mg. In addition, the ZIP4 concentration showed no significant difference from the ZIP4 concentration under the standard diet in spite of the intake of the low-zinc diet and increased to the same level as that under the standard diet.

Accordingly, a ZIP4 concentration increase accelerative action was confirmed by the DHMBA in the supernatant fraction in the low-zinc diet + supernatant fraction group.

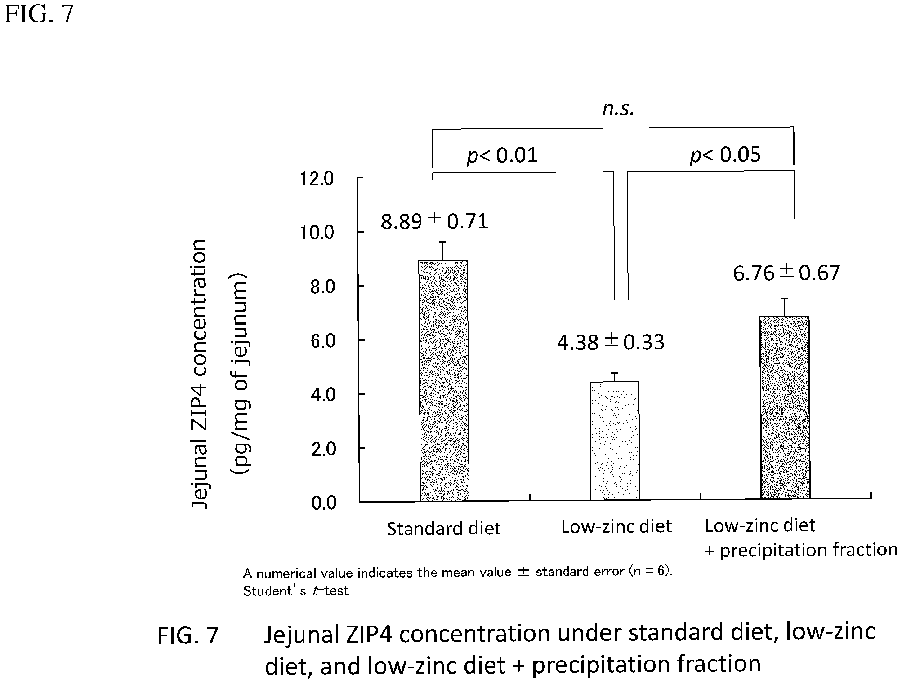

Next, as shown in FIG. 7, the ZIP4 concentration for the low-zinc diet group of 4.38±0.33 pg/mg significantly decreased from the ZIP4 concentration for the standard diet group of 8.89±0.71 pg/mg.

In contrast to this, the low-zinc diet + precipitation fraction group had a ZIP4 concentration of 6.76±0.67 pg/mg, which was significantly higher than the ZIP4 concentration for the low-zinc diet group of 4.38±0.33 pg/mg. In addition, the ZIP4 concentration showed no significant difference from the ZIP4 concentration under the standard diet in spite of the intake of the low-zinc diet and increased to the same level as that under the standard diet. Accordingly, the ZIP4 concentration increase accelerative action was confirmed by the low-zinc diet + precipitation fraction group.

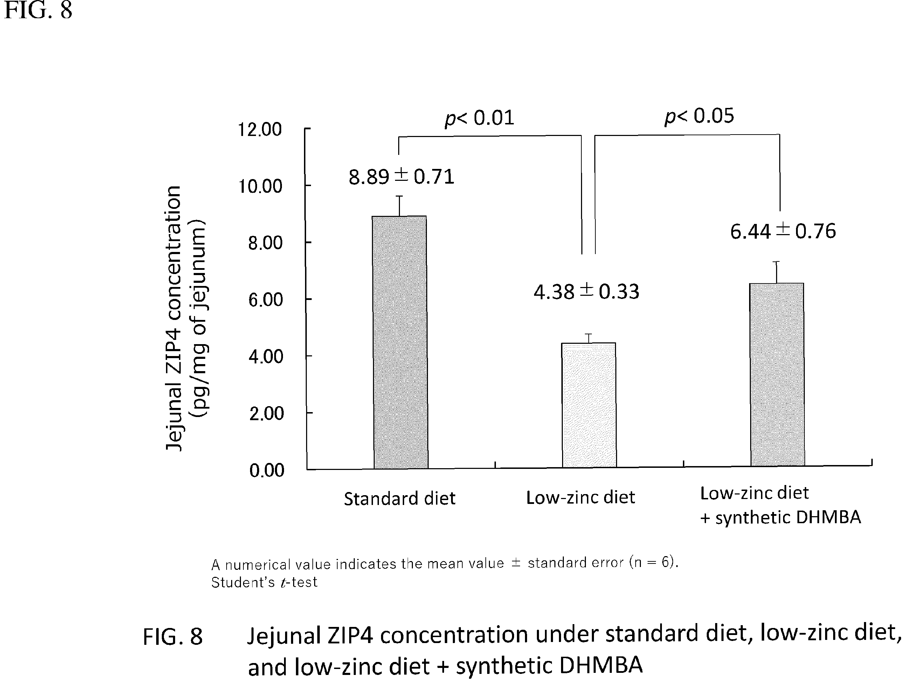

Next, as shown in FIG. 8, the low-zinc diet + synthetic DHMBA fraction group had a ZIP4 concentration of 6.44±0.76 pg/mg, which was significantly higher than the ZIP4 concentration for the low-zinc diet group of 4.38±0.33 pg/mg.

Accordingly, the ZIP4 concentration increase accelerative action by the synthetic DHMBA of the low-zinc diet + synthetic DHMBA group was confirmed.

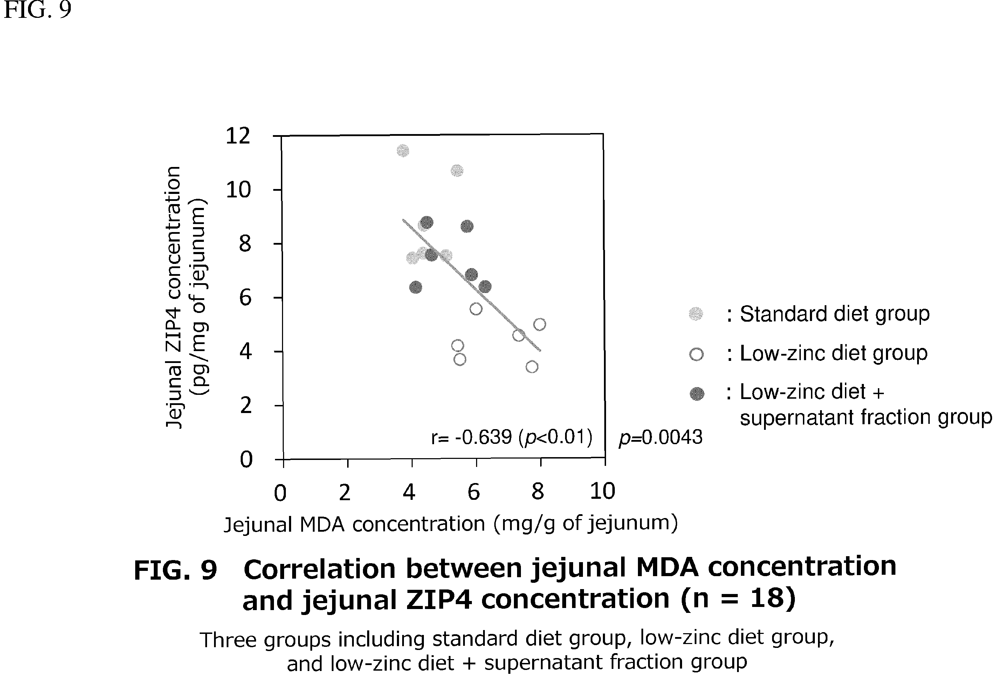

FIG. 9 is a drawing showing the correlation between the jejunal MDA concentration and the jejunal ZIP4 concentration.

As shown in FIG. 9, it could be confirmed that a decrease in the amount of ZIP4 was induced with an increase in the amount of reactive oxygen (an increase in MDA) in the jejunum induced by the intake of the low-zinc diet and that an increase in the amount of ZIP4 was induced with a decrease in the amount of reactive oxygen (a decrease in MDA) in the jejunum by the intake of the DHMBA of the supernatant fraction having an antioxidant function in the low-zinc diet + supernatant fraction group.

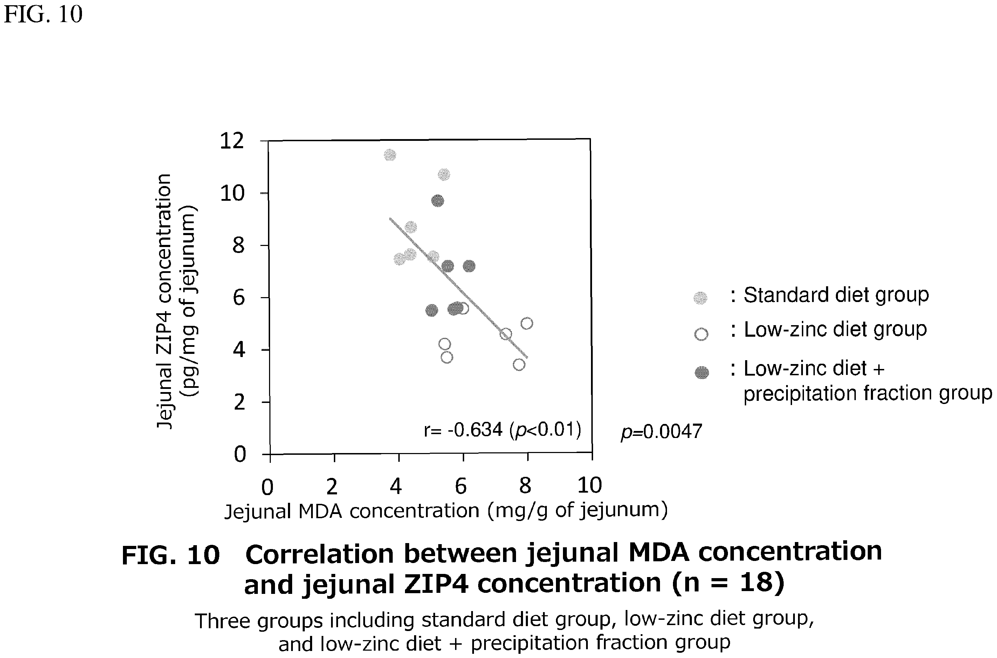

As shown in FIG. 10, a decrease in the amount of ZIP4 was induced with an increase in the amount of reactive oxygen (an increase in MDA) in the jejunum induced by the intake of the low-zinc diet.

Next, it could be confirmed that an increase in the amount of ZIP4 was induced with a decrease in the amount of reactive oxygen (a decrease in MDA) in the jejunum by the intake having an antioxidant function in the low-zinc diet + precipitation fraction group.

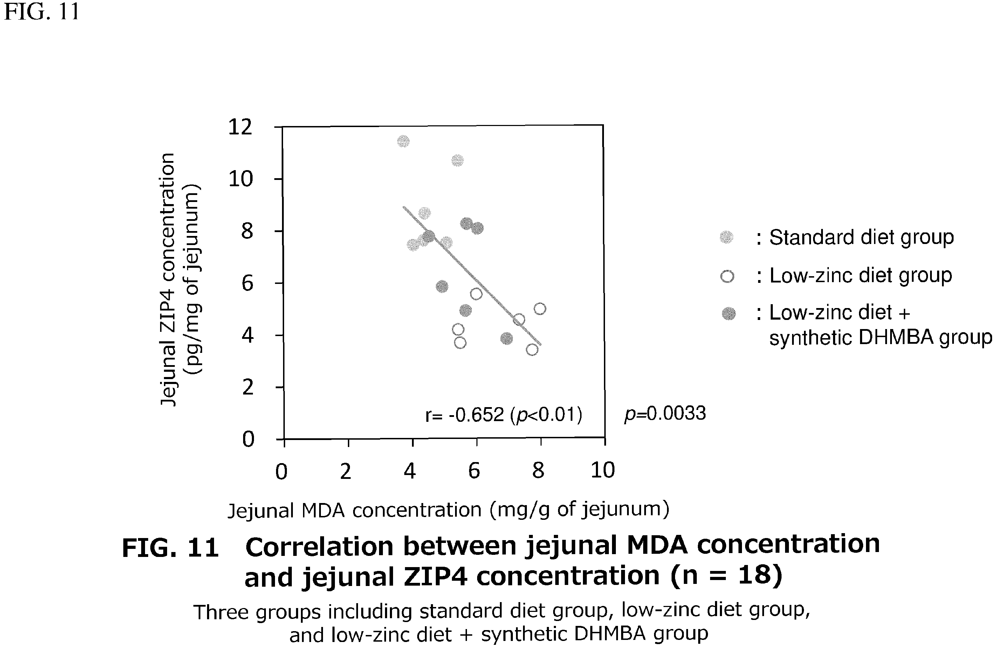

In FIG. 11, it could be confirmed that a decrease in the amount of ZIP4 was induced with an increase in the amount of reactive oxygen (an increase in MDA) in the jejunum induced by the intake of the low-zinc diet, that a decrease in the amount of ZIP4 was induced with an increase in the amount of reactive oxygen (an increase in MDA) in the jejunum induced by the intake of the low-zinc diet, and that an increase in the amount of ZIP4 was induced with a decrease in the amount of reactive oxygen (a decrease in MDA) in the jejunum by the intake of the DHMBA of the synthetic DHMBA fraction having an antioxidant function in the low-zinc diet + synthetic DHMBA fraction group.

As shown in FIG. 9, FIG. 10, and FIG. 11 above, it can be confirmed that there is a close correlation between the jejunal MDA concentration and the jejunal ZIP4 concentration.

That is, as described above, only in corn roots and shoots, ZIP4 did not increase due to oxidative stress, but the increase in ZIP4 due to antioxidant protection had been reported.

However, the present invention further confirmed that while the zinc transporter ZIP4 decreased due to oxidative stress in the jejunum of a rat as a mammal, the ZIP4 increased with an elimination of reactive oxygen (a decrease in MDA) by the administration of DHMB as an antioxidant substance.

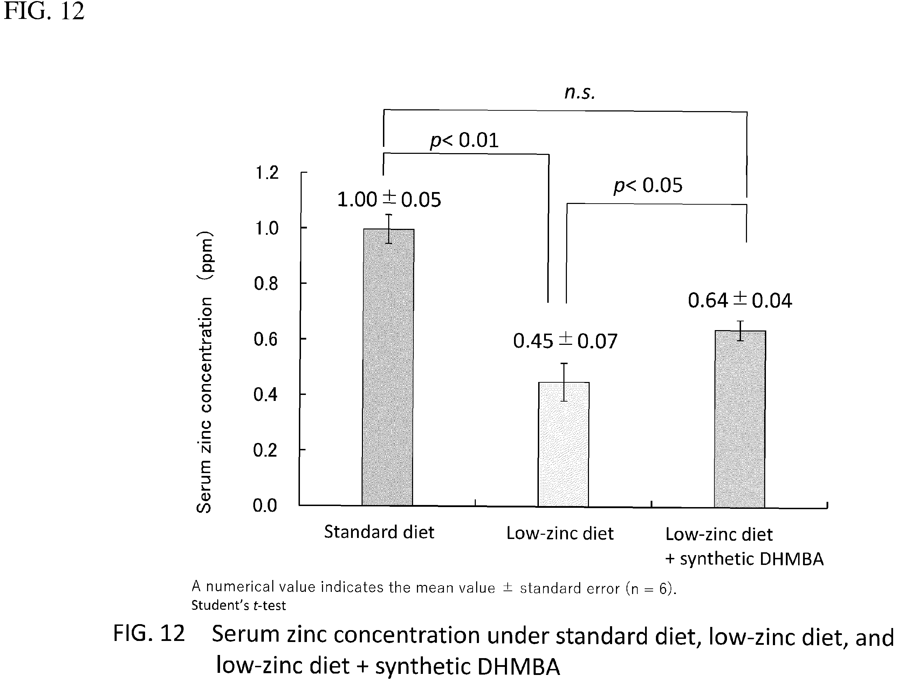

FIG. 12 is a drawing for verifying a change in serum zinc concentration in the standard diet group, the low-zinc diet group, and the low-zinc diet + synthetic DHMBA diet group.

In the synthetic DHMBA fraction, the amount of zinc contained is zero. The amount of zinc taken is the same at the time of the intake in the low-zinc diet group and the time of the intake in the low-zinc diet + synthetic DHMBA diet group.

However, in spite of the intake of the same amount of zinc, the serum zinc concentration for the low-zinc diet + synthetic DHMBA diet group is significantly higher than the serum zinc concentration under the low-zinc diet. This means that the synthetic DHMBA of the synthetic DHMBA fraction increased the amount of zinc absorbed.

Accordingly, as shown in FIG. 12, DHMBA was confirmed to be a functionally involved component that increases the serum zinc concentration in an oxidative stress state.

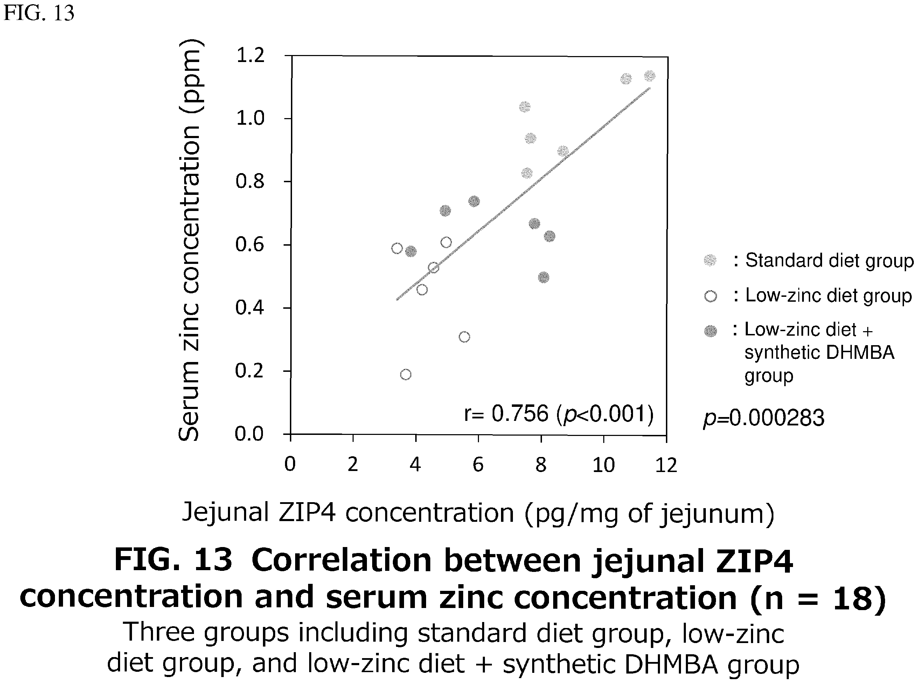

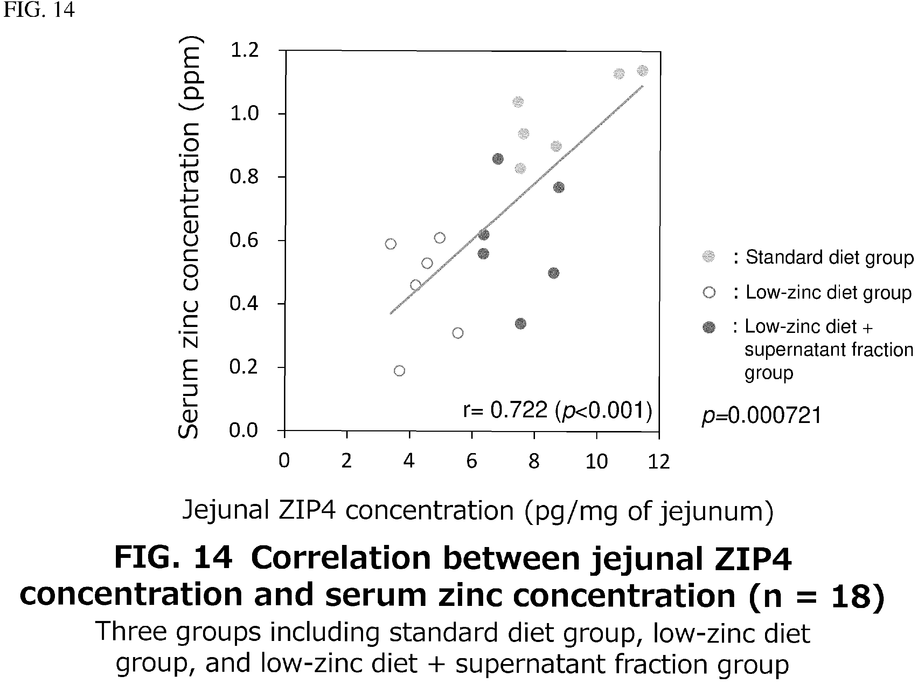

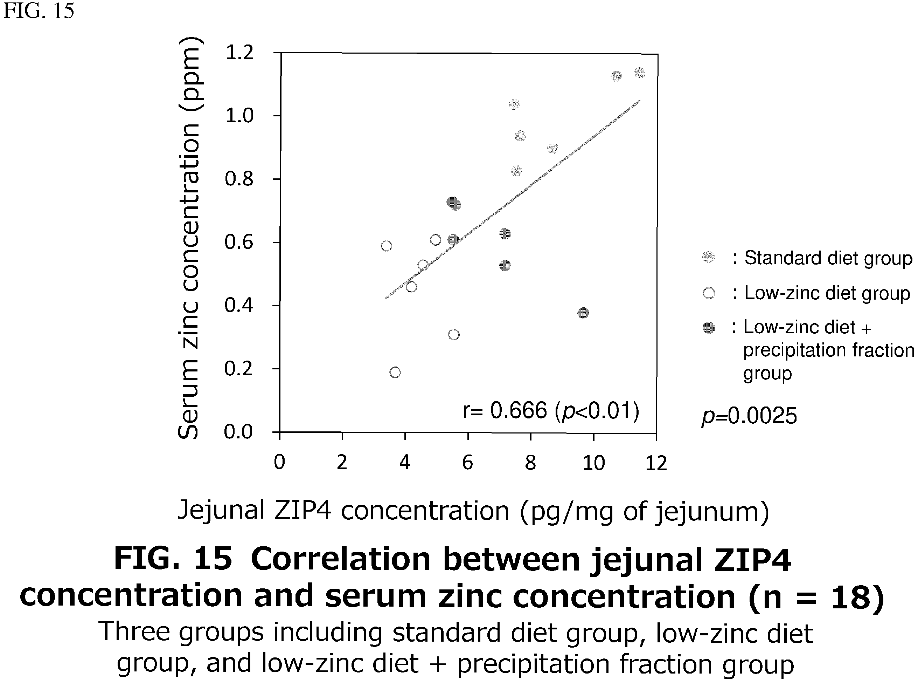

Next, FIG. 13 discusses the correlation between the jejunal ZIP4 concentration and the serum zinc concentration in the standard diet group, the low-zinc diet group, and the low-zinc diet + synthetic DHMBA fraction group. FIG. 14 discusses the correlation between the jejunal ZIP4 concentration and the serum zinc concentration in the standard diet group, the low-zinc diet group, and the low-zinc diet + supernatant fraction group. FIG. 15 discusses the correlation between the jejunal ZIP4 concentration and the serum zinc concentration in the standard diet group, the low-zinc diet group, and the low-zinc diet + precipitation fraction group.

Next, as shown in the correlation between the jejunal ZIP4 concentration and the serum zinc concentration in FIG. 13, the serum zinc concentration showed an increase with an increase in ZIP4 in the jejunum in the three groups including the standard diet group, the low-zinc diet group, and the low-zinc diet + synthetic DHMBA fraction group.

Consequently, it could be confirmed that the serum zinc concentration increased with the increase in ZIP4 in the jejunum by the administration of the synthetic DHMBA of the synthetic DHMBA fraction group, even in the low-zinc diet + synthetic DHMBA fraction group.

In addition, as shown in the correlation between the jejunal ZIP4 concentration and the serum zinc concentration in FIG. 14, the serum zinc concentration showed an increase with an increase in ZIP4 in the jejunum in the three groups including the standard diet group, the low-zinc diet group, and the low-zinc diet + supernatant fraction group.

Consequently, it could be confirmed that the serum zinc concentration increased with the increase in ZIP4 in the jejunum by the administration of DHMBA contained in the supernatant fraction in the low-zinc diet + supernatant fraction group.

As shown in the correlation between the jejunal ZIP4 concentration and the serum zinc concentration in FIG. 15, the serum zinc concentration showed an increase with an increase in ZIP4 in the jejunum in the three groups including the standard diet group, the low-zinc diet group, and the low-zinc diet + precipitation fraction group.

Consequently, it could be confirmed that the serum zinc concentration increased with the increase in ZIP4 in the jejunum by the administration of DHMBA contained in the precipitation fraction in the low-zinc diet + precipitation fraction group.

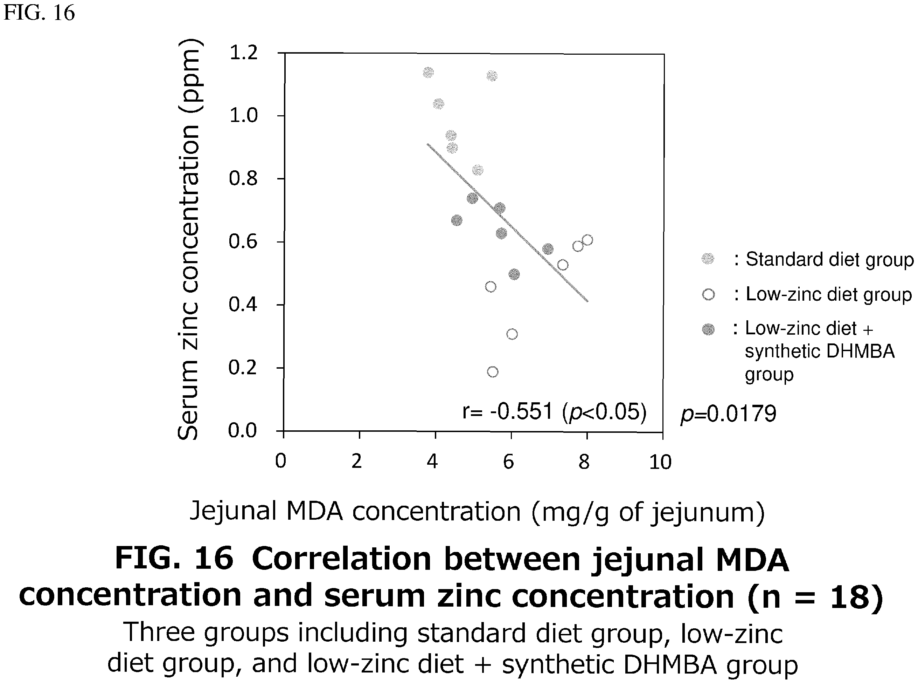

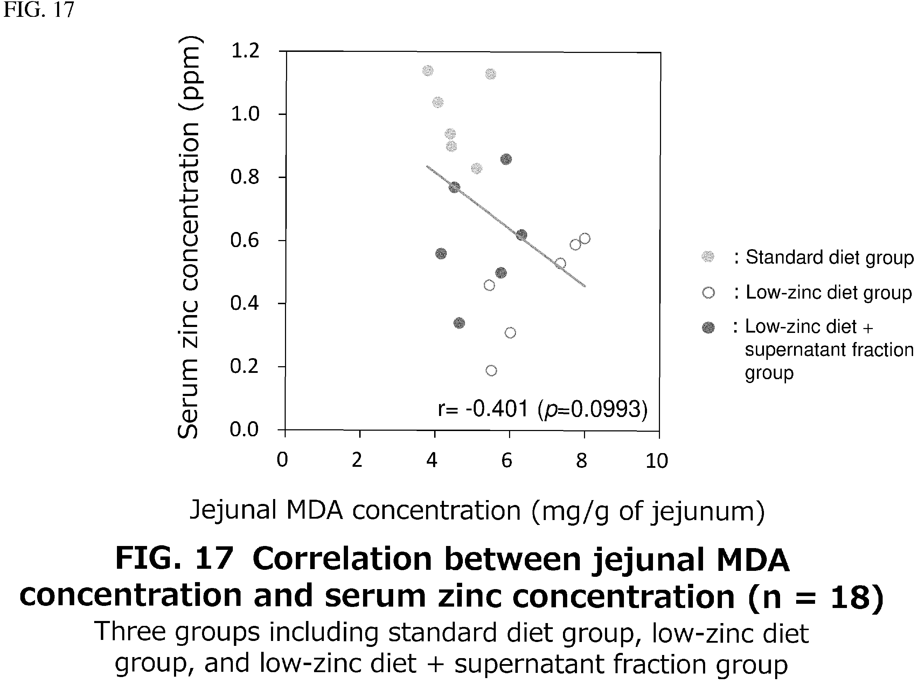

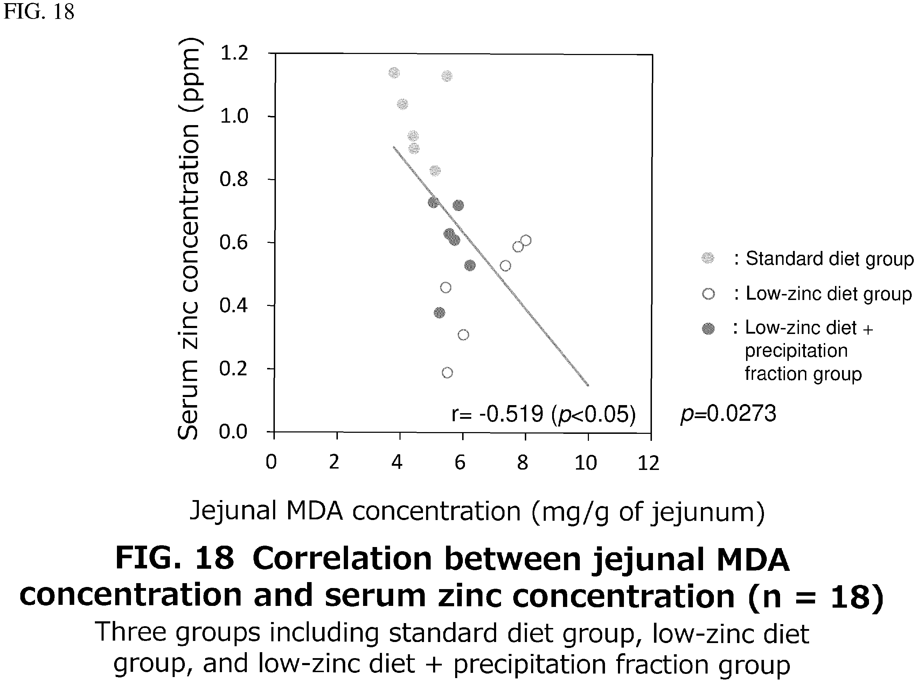

FIG. 16 discusses the correlation between the jejunal MDA concentration and the serum zinc concentration in the standard diet group, the low-zinc diet group, and the low-zinc diet + synthetic DHMBA fraction group. FIG. 17 discusses the correlation between the jejunal MDA concentration and the serum zinc concentration in the standard diet group, the low-zinc diet group, and the low-zinc diet + supernatant fraction group. FIG. 18 discusses the correlation between the jejunal MDA concentration and the serum zinc concentration in the standard diet group, the low-zinc diet group, and the low-zinc diet + precipitation fraction group. From the above FIG. 16, FIG. 17, and FIG. 18, a significant inverse correlation was recognized between the jejunal MDA concentration and the serum zinc concentration.

From FIG. 16, it was confirmed that in the low-zinc diet + synthetic DHMBA group, the serum zinc concentration increased with a decrease in the jejunal MDA concentration (a decrease in hydroxyl radical) by the intake of the synthetic DHMBA. This phenomenon is thought to mean that, as confirmed in FIG. 13 to FIG. 15, the ZIP4 concentration increased with the decrease in reactive oxygen in the jejunum (the decrease in MDA in the jejunum), and consequently, the decrease in the jejunal MDA concentration increased zinc supply to serum.

From FIG. 17, it was confirmed that in the low-zinc diet + supernatant fraction group, the serum zinc concentration increased with a decrease in the jejunal MDA concentration by the intake of the DHMBA of the supernatant fraction.

In the low-zinc diet + precipitation fraction group of FIG. 18, it was confirmed that the serum zinc concentration increased with a decrease in the jejunal MDA concentration by the intake of the DHMBA of the precipitation fraction.

Even in FIG. 17 and FIG. 18, as shown in FIG. 16, the phenomena are thought to mean that, as confirmed in FIG. 13 to FIG. 15, the ZIP4 concentration increased with the decrease in reactive oxygen in the jejunum (the decrease in MDA in the jejunum), and consequently, the decrease in the jejunal MDA concentration increased zinc supply to serum.

## Claims

1. A jejunal 8-OHdG concentration decrease accelerator comprising 3,5-dihydroxy-4-methoxybenzyl alcohol as an active ingredient, the 8-OHdG concentration decrease accelerator having an 8-OHdG concentration decrease accelerative action of decreasing a jejunal 8-OHdG concentration.

2. A jejunal 8-OHdG concentration decrease accelerator comprising 3,5-dihydroxy-4-methoxybenzyl alcohol extracted from oyster meat as an active ingredient, the 8-OHdG concentration decrease accelerator having an 8-OHdG concentration decrease accelerative action of decreasing a jejunal 8-OHdG concentration.

3. A jejunal 8-OHdG concentration decrease accelerator comprising a supernatant fraction as an active ingredient, the supernatant fraction being obtained by stirring an extraction liquid extracted from oyster meat and then taking out a supernatant extract, the 8-OHdG concentration decrease accelerator having an 8-OHdG concentration decrease accelerative action of decreasing a jejunal 8-OHdG concentration.

4. A jejunal 8-OHdG concentration decrease accelerator comprising a precipitation fraction as an active ingredient, the precipitation fraction being precipitated after stirring an extraction liquid extracted from oyster meat, the 8-OHdG concentration decrease accelerator having an 8-OHdG concentration decrease accelerative action of decreasing a jejunal 8-OHdG concentration.

5. A jejunal 8-OHdG concentration decrease accelerator comprising synthesized 3,5-dihydroxy-4-methoxybenzyl alcohol as an active ingredient, the 8-OHdG concentration decrease accelerator having an 8-OHdG concentration decrease accelerative action of decreasing a jejunal 8-OHdG concentration.

6. A jejunal MDA concentration decrease accelerator comprising 3,5-dihydroxy-4-methoxybenzyl alcohol as an active ingredient, the MDA concentration decrease accelerator having an MDA concentration decrease accelerative action of decreasing a jejunal MDA concentration.

7. A jejunal MDA concentration decrease accelerator comprising 3,5-dihydroxy-4-methoxybenzyl alcohol extracted from oyster meat as an active ingredient, the MDA concentration decrease accelerator having an MDA concentration decrease accelerative action of decreasing a jejunal MDA concentration.

8. A jejunal MDA concentration decrease accelerator comprising a supernatant fraction as an active ingredient, the supernatant fraction being obtained by stirring an extraction liquid extracted from oyster meat and then taking out a supernatant extract, the MDA concentration decrease accelerator having an MDA concentration decrease accelerative action of decreasing a jejunal MDA concentration.

9. A jejunal MDA concentration decrease accelerator comprising a precipitation fraction as an active ingredient, the precipitation fraction being precipitated after stirring an extraction liquid extracted from oyster meat, the MDA concentration decrease accelerator having an MDA concentration decrease accelerative action of decreasing a jejunal MDA concentration.

10. A jejunal MDA concentration decrease accelerator comprising synthesized 3,5-dihydroxy-4-methoxybenzyl alcohol as an active ingredient, the MDA concentration decrease accelerator having an MDA concentration decrease accelerative action of decreasing a jejunal MDA concentration.

11. A jejunal ZIP4 concentration increase accelerator comprising 3,5-dihydroxy-4-methoxybenzyl alcohol as an active ingredient, the ZIP4 concentration increase accelerator having a ZIP4 concentration increase accelerative action of increasing a jejunal ZIP4 concentration.

12. A jejunal ZIP4 concentration increase accelerator comprising 3,5-dihydroxy-4-methoxybenzyl alcohol extracted from oyster meat as an active ingredient, the ZIP4 concentration increase accelerator having a ZIP4 concentration increase accelerative action of increasing a jejunal ZIP4 concentration.

13. A jejunal ZIP4 concentration increase accelerator comprising a supernatant fraction as an active ingredient, the supernatant fraction being obtained by stirring an extraction liquid extracted from oyster meat and then taking out a supernatant extract, the ZIP4 concentration increase accelerator having a ZIP4 concentration increase accelerative action of increasing a jejunal ZIP4 concentration.

14. A jejunal ZIP4 concentration increase accelerator comprising a precipitation fraction as an active ingredient, the precipitation fraction being precipitated after stirring an extraction liquid extracted from oyster meat, the ZIP4 concentration increase accelerator having a ZIP4 concentration increase accelerative action of increasing a jejunal ZIP4 concentration.

15. A jejunal ZIP4 concentration increase accelerator comprising synthesized 3,5-dihydroxy-4-methoxybenzyl alcohol as an active ingredient, the ZIP4 concentration increase accelerator having a ZIP4 concentration increase accelerative action of increasing a jejunal ZIP4 concentration.

16. A serum zinc concentration increase inducer comprising 3,5-dihydroxy-4-methoxybenzyl alcohol as an active ingredient, the serum zinc concentration increase inducer having an action of inducing an increase in serum zinc concentration.

17. A serum zinc concentration increase inducer comprising 3,5-dihydroxy-4-methoxybenzyl alcohol extracted from oyster meat as an active ingredient, the serum zinc concentration increase inducer having an action of inducing an increase in serum zinc concentration.

18. A serum zinc concentration increase inducer comprising synthesized 3,5-dihydroxy-4-methoxybenzyl alcohol as an active ingredient, the serum zinc concentration increase inducer having an action of inducing an increase in serum zinc concentration.

19. A serum zinc concentration increase inducer comprising a supernatant fraction containing DHMBA as an active ingredient, the supernatant fraction being obtained by stirring an extraction liquid extracted from oyster meat and then taking out a supernatant extract, the serum zinc concentration increase inducer having an action of inducing an increase in serum zinc concentration.

20. A serum zinc concentration increase inducer comprising a precipitation fraction containing DHMBA as an active ingredient, the precipitation fraction being precipitated after stirring an extraction liquid extracted from oyster meat, the serum zinc concentration increase inducer having an action of inducing an increase in serum zinc concentration.
